# EUROPEAN PATENT SPECIFICATION

(11) **EP 3 790 020 B1**
(45) Date of publication and mention of the grant of the patent: **20.05.2026**
(21) Application number: 20780002.0
(22) Date of filing: 26.02.2020
(51) Int. Cl.: G06Q 10/0639, G06Q 20/02, G06Q 20/08, G06Q 20/36, G06Q 20/40, G06Q 30/04, G06Q 30/06, G16H 40/20, G16H 70/20, G16H 80/00

(54) **MEDICAL SERVICE MANAGEMENT METHOD AND DEVICE**
VERFAHREN UND VORRICHTUNG ZUR VERWALTUNG MEDIZINISCHER DIENSTLEISTUNGEN
PROCÉDÉ ET DISPOSITIF DE GESTION DE SERVICE MÉDICAL

(30) Priority: 27.03.2019 CN 201910237975
(43) Date of publication of application: 10.03.2021
(73) Proprietor: Launch Tech Co., Ltd, Longgang District Shenzhen Guangdong 518000 (CN)
(72) Inventor: LIU, Xin, Shenzhen, Guangdong 518000 (CN)
(74) Representative: Mathys & Squire
(86) International application number: PCT/CN2020/076755
(87) International publication number: WO 2020/192342

(56) References cited:
- CN-A- 107 292 597
- CN-A- 107 863 134
- CN-A- 108 053 868
- CN-A- 108 053 868
- CN-A- 108 550 390
- CN-A- 109 326 340
- CN-A- 109 979 580
- US-A1- 2017 039 330
- US-A1- 2017 372 300
- US-A1- 2018 060 496
- US-A1- 2018 330 348
- US-A1- 2019 035 508

## Description

### TECHNICAL FIELD

This disclosure relates to the field of computer technology, and particularly to a method for medical service management and related devices.

### BACKGROUND

As medical informatization advanced rapidly, electronicization of patients' medical data has gradually developed, which can speed up the process of various registration procedures for patients when patients seek medical treatments. The existing medical system needs to rely on medical staffs to uniformly manage information of patients such as medical treatment process information, medical fee, treatment course, and so on. The above information is, however, completely managed by hospitals, resulting in low patient satisfaction with medical services provided. Therefore, achieving transparency of the medical services and improving the quality of the medical services have become an urgent problem to be solved.

At present, management concepts of hospitals are obsolete, and working intensity of medical staffs is relatively large while salaries of the medical staffs are relatively low. Moreover, the medical services are difficult to quantify. Professional qualities of medical staffs play a crucial role in their work enthusiasm and service quality. However, in practice, there is no reasonable evaluation on workloads of the medical staffs. In addition, complex charging standards and payment procedures have seriously affected the patient's medical experience, and the treatment is relatively inefficient.

Relevant prior art disclosures include the documents US2018330348A1, and US2018060496A1.

### SUMMARY

The invention is defined by the independent claims. According to implementations, a method for medical service management and related devices are provided, which can realize transparency of medical services, accurately evaluate workloads of medical staffs, and simplify procedures for patients to pay medical fee.

A method for medical service management is provided. The method is applicable to a blockchain node device and includes the following. An appointment request from a user is received, where the appointment request includes a user identity. Medical service information is recommended according to the appointment request, where the medical service information includes recommended medical-staff information, recommended medical service type information, and recommended drug information. A medical service scheme is determined according to the medical service information. A medical-fee payment contract is generated according to the medical service scheme. In response to receiving from the user a confirmation message of the medical-fee payment contract, according to the medical-fee payment contract, medical fee is transferred from a digital wallet of the user to at least one digital wallet of at least one related party of the medical service scheme.

Advantageous effects: according to the implementations of the present disclosure, by storing medical service records in blockchain, the transparency of the medical services can be realized, the workloads of medical staffs can be accurately evaluated, and the procedures for patients to pay medical fee can be effectively simplified.

### BRIEF DESCRIPTION OF THE DRAWINGS

FIG. 1 is an architecture diagram illustrating a system for medical service management according to some implementations.
FIG. 2 is a schematic flowchart illustrating a method for medical service management according to some implementations.
FIG. 3 is a schematic structural diagram illustrating a blockchain node device according to some implementations.
FIG. 4 is a schematic structural diagram illustrating hardware of a blockchain node device according to some implementations.

### DETAILED DESCRIPTION

Hereinafter, technical solutions of implementations of the present disclosure will be described below with reference to the drawings of the present disclosure.

At present, management concepts of hospitals are obsolete, and working intensity of medical staffs is relatively large while salaries of the medical staffs are relatively low. Information of patients such as medical fee, treatment course, and so on is completely managed by hospitals, resulting in low patient satisfaction with medical services provided. Therefore, achieving transparency of the medical services and improving the quality of the medical services have become an urgent problem to be solved. To this end, implementations of the present disclosure provide a method for medical service management and related devices. By storing medical service records in the blockchain, the transparency of the medical services can be achieved, workloads of medical staffs can be accurately evaluated, and procedures for patients to pay medical fee can be effectively simplified.

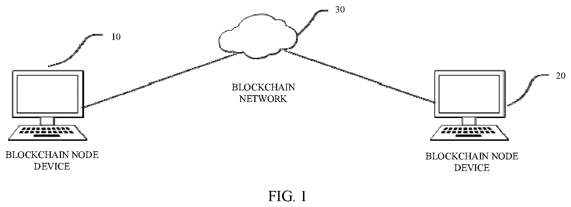

FIG. 1 is a system architecture diagram illustrating a system for medical service management according to some implementations. As illustrated in FIG. 1, the system includes a blockchain node device 10, a blockchain node device 20, and a blockchain network 30. Multiple blockchain node devices can communicate and couple with each other through the blockchain network 30, and the manner of communication connection between blockchain node devices and the blockchain network is not limited in the present disclosure.

In implementations of the present disclosure, the blockchain node device may be an electronic device used by a user or a hospital party. The blockchain node device can store user identity information or medical-staff information in the blockchain network. In response to receiving an appointment request from a user, the blockchain node device recommends medical service information to the user according to the appointment request. After diagnosis and treatment, a medical service scheme and a medical-fee payment contract are confirmed according to the medical service information. The blockchain node device transfers, according to the medical-fee payment contract, medical fee from a digital wallet of the user to at least one digital wallet of at least one related party of the medical service scheme.

In implementations of the present disclosure, an identity of a user of the blockchain node device needs to be verified, and the entire medical process is recorded in the blockchain network, which can ensure transparency of the entire medical process, accurately evaluate the workloads of medical staffs, and simplify the procedures for users to pay medical fee.

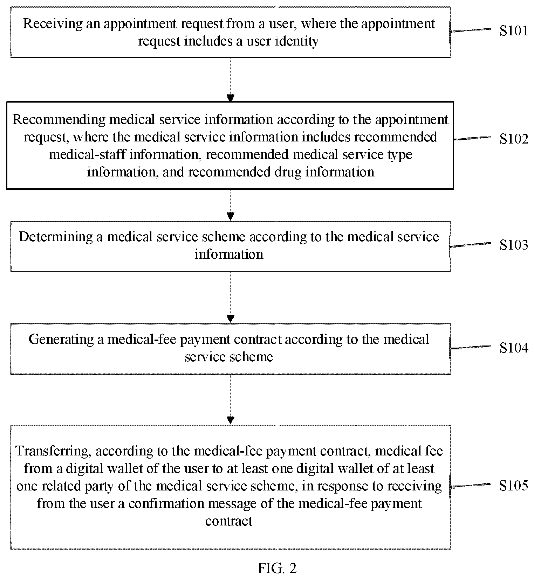

FIG. 2 is a schematic flowchart illustrating a method for medical service management according to some implementations. As illustrated in FIG. 2, the method includes the following.

At S101, an appointment request from a user is received, where the appointment request includes a user identity.

In the implementation of the present disclosure, the foregoing blockchain node device is a blockchain node device capable of accessing the Internet. The blockchain node device includes, but is not limited to, a server, a portable tablet computer, a notebook computer, a desktop computer, a smart phone, an on-board terminal, an on-board diagnostic (OBD) device, a wearable bracelet, a wearable watch, a headphone, and other electronic products. It can be understood that, the blockchain node device and the blockchain network are not limited in the present disclosure.

In one implementation, before receiving the appointment request from the user, an identity of a medical staff using the blockchain node device needs to be verified. If authentication performed by an authorized node on the identity passes, it indicates that the qualification of the medical staff (e.g., a medical qualification, etc.) is reliable. Then the blockchain node device receives the appointment request from the user. The authorized node herein may be one of a plurality of blockchain node devices representing different official authorities or trusted authorities in the blockchain network, or a trusted blockchain node device trusted by multiple official authorities.

In one implementation, the authorized node authenticates qualifications of medical staffs, and all medical staffs certified by the authorized node have the qualification of legitimate medical practice. Only a doctor who actually works in a hospital can pass the above authentication, which can avoid occurrence of nominal practice.

In one implementation, according to the appointment request, a time for which the user wants to make an appointment can be known. Then according to specific schedule of a reserved hospital, relevant information is sent to a blockchain node device corresponding to the user.

As an example, an appointment request which requests for a visit on Thursday is received by a doctor in the reserved hospital. After checking a schedule, the doctor learns that he would not be sitting on Thursday. In this case, scheduling information is sent to the user, and the user is suggested to change a visit time.

In one implementation, personal information of the user is verified according to the user identity.

In one implementation, according to the user identity, at least one of personal medical insurance information and personal medical record information is obtained. A verification result of at least one of the personal medical insurance information and the personal medical record information is obtained.

For example, because procedures such as registration do not require identity verification, ticket touts can monopolize consultation opportunities of an expert by buying out registration opportunities of the expert in advance, which makes many users who actually need medical treatment lose the registration opportunities. However, in this disclosure, the appointment request needs to be sent by the user himself. Even if the ticket tout can send multiple appointment requests, because the ticket tout is not a user who actually seeks a medical treatment, the ticket tout cannot obtain multiple medical treatment opportunities. In this disclosure, personal medical insurance information of the user can be used to check whether the user is a ticket tout, or to check whether there is any bad behavior such as occupying reservations and wasting medical opportunities.

In one implementation, the user identity information is verified by the authorized node. The authorized node can perform verification on the user identity information and add authentication. If authentication for the user performed by the authorized node passes, it indicates that personal medical insurance information and personal medical record information involved are reliable. A digital identity of the user can represent the user himself.

In one implementation, before receiving the appointment request from the user, information of at least one related party participating in a medical service process is confirmed, and a sharing ratio for each of the at least one related party is determined.

In one implementation, a registration request of the at least one related party is received, where the at least one related party at least includes one of a doctor, a nurse, an auxiliary person, a medical-equipment provider, and a medical-equipment manager. According to the registration request, for each of the at least one related party, a medical service that the related party needs to participate in is determined. For each of the at least one related party, a sharing ratio for the related party is determined according to the medical service that the related party needs to participate in.

At S102, medical service information is recommended according to the appointment request, where the medical service information includes recommended medical-staff information, recommended medical service type information, and recommended drug information.

In one implementation, medical record information of the user is obtained according to the appointment request. By sorting one or more pieces of medical-staff information according to the medical record information of the user, one or more groups of sorted medical-staff information are obtained. One group of medical-staff information selected by the user from the one or more groups of sorted medical-staff information is received. The recommended medical-staff information is determined, where the recommended medical-staff information includes following information of a medical staff: an identity, department information, skill certification information, length of service information, occupation information, and medical experience information.

As an example, according to the personal medical record information of the user, it can be known that the user had a "chest pain" after suffering a fracture. To avoid misdiagnosis, medical-staff information of a cardiology department is first recommended to the user, and the user is arranged to receive medical services in the cardiology department (e.g., an electrocardiogram diagnosis service and other medical services). Medical-staff information of an orthopedics department and related medical services are further recommended after screening of each diagnosis of the cardiology department is completed.

As another example, information such as a desired distance to a hospital and length of service can be set by the user. The blockchain node device searches all pieces of medical-staff information according to conditions set by the user. The blockchain node device selects and displays medical-staff information that satisfies both conditions of the set desired hospital distance and the set length of service, or selects and displays medical-staff information that satisfies one of conditions of the set desired hospital distance and the set length of service for the user to choose.

At S103, a medical service scheme is determined according to the medical service information.

The medical service scheme herein may include the number of medical treatments to the user performed by medical staffs, the number of different medical services experienced, the types of drugs, and medication course.

In one implementation, after a recommended medical staff completes the diagnosis of the use, the recommended medical staff arranges relevant auxiliary examination or relevant physical therapy for the user according to use's condition.

For example, after an X-ray examination, the user is diagnosed with a fracture. According to patient's condition, Dr. Wang (i.e., the recommended medical staff) performs reset and fixed treatments for the user, arranges treatments for weekly functional exercise for the user, and instructs the user to take drug A three times a day.

At S104, a medical-fee payment contract is generated according to the medical service scheme.

The medical-fee payment contract herein may include medical fee corresponding to each item of the medical service scheme.

In one implementation, after receiving the medical-fee payment contract, the medical service scheme involved in the contract can be seen by the user.

For example, the doctor performs orthopedic treatment for the user during a treatment. The medical service scheme can help the user learn the name of an obtained treatment and relevant drug information during the entire orthopedic treatment. With the help of the medical service scheme, the user can view his own records of the orthopedic treatment, the cost of materials used in a treatment process, and information on drugs such as safflower oil needed for a subsequent treatment course.

In one implementation, the user confirms whether the contents of the medical-fee payment contract are true, to ensure that a hospital party does not add redundant medical procedures. Before paying relevant fee, the user is required to make sure that medical services experienced are the same as those stated in the medical-fee payment contract. If the medical services experienced are different from those stated in the medical-fee payment contract, the user can check the relevant records.

In one implementation, if the user confirms that the medical services experienced are consistent with medical services provided by the hospital party, confirmation information indicating that the medical service scheme of the hospital party is correct is sent to the hospital party.

In one implementation, if the user confirms that the medical services provided by the hospital party are inconsistent with the medical services experienced and the medical service scheme of the hospital party is inconsistent with the actual medical service scheme, a medical service scheme updated by the user is sent to the hospital party, to remind the blockchain node device to further verify the updated medical service scheme.

In one implementation, after the user proves that he has experienced the above medical services, a medical service record is generated in response to receiving a confirmation request. That is, the medical service record should also be confirmed by the user. In this implementation, the patient can compare the medical service record with the medical-fee payment contract to decide whether to fulfill the medical-fee payment contract.

At S105, in response to receiving from the user a confirmation message of the medical-fee payment contract, according to the medical-fee payment contract, medical fee is transferred from a digital wallet of the user to at least one digital wallet of at least one related party of the medical service scheme.

After the hospital party provides the medical services for the user, the medical-fee payment contract needs to be fulfilled, and fee corresponding to each of the medical services is transfer to a digital wallet of a medical staff providing the medical service. The confirmation message is a message for confirming that the medical service scheme is correct, or a message for confirming of an updated medical service scheme.

In one implementation, the confirmation message is a message for confirming of an updated medical service scheme. A new medical-fee payment contract is generated in response to an updating message. In response to receiving from the user a confirmation message indicating that the new medical-fee payment contract is correct, the new medical-fee payment contract is fulfilled.

In one implementation, for each of the at least one related party of the medical service scheme, a sharing ratio corresponding to the related party is obtained. For each of the at least one related party of the medical service scheme, according to the sharing ratio obtained, the medical fee is transferred to a digital wallet of the related party of the medical service scheme.

In one implementation, after the user pays the medical fee, the medical fee is directly distributed in a certain proportion (i.e., based on sharing ratios) to all related parties participating in the medical service scheme.

As an example, the user obtains a treatment in the orthopedics department and pays 200 yuan for the treatment. Sharing ratios of the fee paid by the user for example are 30% for a doctor, 30% for a pharmacy manager, 10% for a nurse, 10% for a rehabilitation assistant, and 20% for an equipment provider, respectively. Accordingly, digital wallets of the doctor and the pharmacy manager each increase by 60 yuan, digital wallets of the nurse and the rehabilitation assistant each increase by 20 yuan, and a digital wallet of the equipment provider increases by 40 yuan.

For example, Xiao Liu is assigned to dispense medicine for the user. If a sharing ratio of Xiao Liu is 5%, when the digital wallet of the pharmacy manager receives a corresponding payment, a digital wallet of Xiao Liu increases by 10 yuan according to the predetermined sharing ratio.

In one implementation, a fee-sharing party may be designated by the user first among the related parties of the medical service scheme. After the medical fee is paid, the medical fee is first transferred into a digital wallet of the designated fee-sharing party, and then relevant fee is distributed by the designated fee-sharing party to other related parties.

As an example, the designated fee-sharing party is an equipment provider providing the medical services experienced by the user. According to sharing ratios (i.e., 30% for a doctor, 30% for a pharmacy manager, 10% for a nurse, 10% for a rehabilitation assistant, and 20% for an equipment provider), the designated fee-sharing party transfers remaining payment after deducting his own part (i.e., 20%) to digital wallets of other related parties.

As another example, a specific fee-sharing party is assigned. The fee-sharing party does not provide the medical services experienced by the user. After the medical fee paid by the user is transferred to an account of the fee-sharing party (i.e., a digital wallet of the fee-sharing party), the fee-sharing party deducts his own proportion (i.e., 1% for management), and transfers, according to the sharing ratios, remaining payment to the at least one digital wallet of the at least one related party of the medical service scheme.

According to the method for medical service management of the implementations of the present disclosure, an identity of a communication object can be verified, and the medical fee paid by the user can be distributed to the at least one related party participating in the medical services. By adopting the scheme of the present disclosure, medical opportunities of users can be guaranteed, workloads of medical staffs can be accurately evaluated, and procedures for the user to pay medical fee can be effectively simplified.

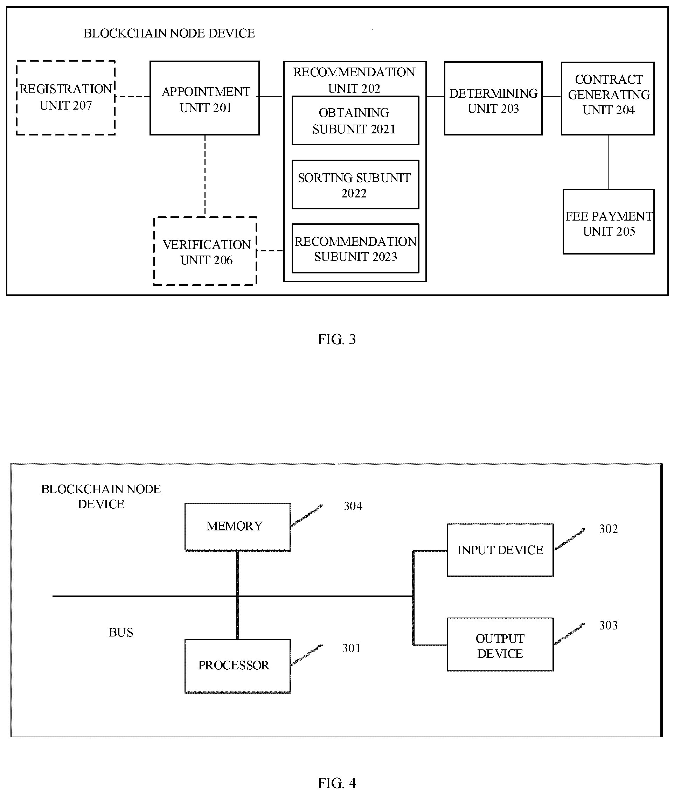

FIG. 3 is a schematic structural diagram illustrating a blockchain node device according to some implementations. As illustrated in FIG. 3, the blockchain node device includes an appointment unit 201, a recommendation unit 202, a determining unit 203, a contract generating unit 204, and a fee payment unit 205. In one implementation, the blockchain node device further includes a verification unit 206. In one implementation, the blockchain node device further includes a registration unit 207.

The appointment unit 201 is configured to receive an appointment request from a user, where the appointment request includes a user identity. The recommendation unit 202 is configured to recommend medical service information according to the appointment request, where the medical service information includes recommended medical-staff information, recommended medical service type information, and recommended drug information. The determining unit 203 is configured to determine a medical service scheme according to the medical service information. The contract generating unit 204 is configured to generate a medical-fee payment contract according to the medical service scheme. The fee payment unit 205 is configured to transfer, according to the medical-fee payment contract, medical fee from a digital wallet of the user to at least one digital wallet of at least one related party of the medical service scheme, in response to receiving from the user a confirmation message of the medical-fee payment contract.

In one implementation, the blockchain node device further includes a verification unit 206. The verification unit 206 is configured to verify personal information of the user according to the user identity.

In one implementation, the verification unit 206 is configured to: obtain, according to the user identity, at least one of personal medical insurance information and personal medical record information; and obtain a verification result of at least one of the personal medical insurance information and the personal medical record information.

In one implementation, the blockchain node device further includes a registration unit 207. The registration unit 207 is configured to receive a registration request of the at least one related party, where the at least one related party at least includes one of a doctor, a nurse, an auxiliary person, a medical-equipment provider, and a medical-equipment manager. The registration unit 207 is configured to determine, for each of the at least one related party, according to the registration request, a medical service that the related party needs to participate in. The registration unit 207 is configured to determine, for each of the at least one related party, a sharing ratio for the related party according to the medical service that the related party needs to participate in.

In one implementation, the recommendation unit 202 includes an obtaining subunit 2021, a sorting subunit 2022, and a recommendation subunit 2023. The obtaining subunit 2021 is configured to obtain medical record information of the user according to the appointment request. The sorting subunit 2022 is configured to obtain one or more groups of sorted medical-staff information by sorting one or more pieces of medical-staff information according to the medical record information of the user. The recommendation subunit 2023 is configured to receive one group of medical-staff information selected by the user from the one or more groups of sorted medical-staff information, and determine the recommended medical-staff information, where the recommended medical-staff information includes following information of a medical staff: an identity, department information, skill certification information, length of service information, occupation information, and medical experience information.

In one implementation, the fee payment unit 205 is configured to obtain, for each of the at least one related party of the medical service scheme, a sharing ratio corresponding to the related party. The fee payment unit 205 is configured to transfer, for each of the at least one related party of the medical service scheme, according to the sharing ratio obtained, the medical fee to a digital wallet of the related party of the medical service scheme.

For details of the appointment unit 201, the recommendation unit 202, the determining unit 203, the contract generating unit 204, the fee payment unit 205, the verification unit 206, and the registration unit 207, reference may be made to the related descriptions of the method for medical service management in the foregoing method implementations described in conjunction with FIG. 2, which will not be described in further detail herein.

According to the blockchain node device of the implementations of the present disclosure, an identity of a communication object can be verified, and the medical fee paid by the user can be distributed to the at least one related party participating in the medical services. By adopting the scheme of the present disclosure, medical opportunities of users can be guaranteed, workloads of medical staffs can be accurately evaluated, and procedures for the user to pay medical fee can be effectively simplified.

FIG. 4 is a schematic structural diagram illustrating hardware of a blockchain node device according to some implementations. As illustrated in FIG. 4, the blockchain node device includes a processor 301, an input device 302, an output device 303, and a memory 304. The processor 301, the input device 302, the output device 303, and the memory 304 are coupled with each other via a bus.

The memory may include, but is not limited to, a random access memory (RAM), a read-only memory (ROM), an erasable programmable read-only memory (EPROM), or a compact disc read-only memory (CD-ROM), which is configured to store related instructions and data.

The input device is configured to input data and/or signals. The output device is configured to output data and/or signals. The output device and the input device may be independent devices, or may be an integrated device.

The processor may include one or more processors. For example, the processor includes one or more central processing units (CPU). When the processor is embodied as a processor including a CPU, the CPU may be a single-core CPU or a multi-core CPU.

The memory is configured to store program codes and data of network devices.

The processor is configured to call the program codes and the data in the memory and execute the following operations. The input device is controlled to receive an appointment request from a user, where the appointment request includes a user identity. Medical service information is recommended according to the appointment request, where the medical service information includes recommended medical-staff information, recommended medical service type information, and recommended drug information. A medical service scheme is determined according to the medical service information. A medical-fee payment contract is generated according to the medical service scheme. In response to receiving from the user a confirmation message of the medical-fee payment contract, the output device is controlled to transfer, according to the medical-fee payment contract, medical fee from a digital wallet of the user to at least one digital wallet of at least one related party of the medical service scheme.

In one implementation, after controlling the input device to receive the appointment request from the user, the processor is further configured to verify personal information of the user according to the user identity.

In one implementation, the personal information includes at least one of personal medical insurance information and personal medical record information. The processor configured to verify the personal information of the user according to the user identity is configured to: obtain, according to the user identity, at least one of the personal medical insurance information and the personal medical record information; and obtain a verification result of at least one of the personal medical insurance information and the personal medical record information.

In one implementation, prior to controlling the input device to receive the appointment request from the user, the processor is further configured to: control the input device to receive a registration request of the at least one related party, where the at least one related party at least includes one of a doctor, a nurse, an auxiliary person, a medical-equipment provider, and a medical-equipment manager; for each of the at least one related party, according to the registration request, determine a medical service that the related party needs to participate in; for each of the at least one related party, determine a sharing ratio for the related party according to the medical service that the related party needs to participate in.

In one implementation, the processor configured to recommend the medical service information according to the appointment request is configured to: obtain medical record information of the user according to the appointment request; obtain one or more groups of sorted medical-staff information by sorting one or more pieces of medical-staff information according to the medical record information of the user; receive one group of medical-staff information selected by the user from the one or more groups of sorted medical-staff information; and determine the recommended medical-staff information, where the recommended medical-staff information includes following information of a medical staff: an identity, department information, skill certification information, length of service information, occupation information, and medical experience information.

In one implementation, the confirmation message is a message for confirming that the medical service scheme is correct.

In one implementation, the confirmation message is a message for confirming of an updated medical service scheme.

In one implementation, the processor configured to control the output device to transfer, according to the medical-fee payment contract, the medical fee from the digital wallet of the user to the at least one digital wallet of the at least one related party of the medical service scheme is configured to: for each of the at least one related party of the medical service scheme, obtain a sharing ratio corresponding to the related party; for each of the at least one related party of the medical service scheme, according to the sharing ratio obtained, transfer the medical fee to a digital wallet of the related party of the medical service scheme.

It can be understood that, FIG. 4 merely illustrates a simplified design of the blockchain node device. In practice, the blockchain node device may further include other necessary components. Other necessary components include, but are not limited to, any number of input/output devices, processors, controllers, memories, etc. Blockchain node devices that can implement the implementations of the present disclosure shall all be encompassed within the protection of the present disclosure.

The computer readable storage medium may be an internal storage module of the terminal device according to any of the foregoing implementations, such as a hard disk or a memory of the terminal device. The computer readable storage medium may also be an external storage device of the terminal device, for example, a plug-in hard disk equipped on the terminal device, a smart media card (SMC), a secure digital (SD) card, a flash card, etc. Further, the computer readable storage medium may also include both an internal storage module of the terminal device and an external storage device. The computer readable storage medium is used to store computer programs and other programs and data required by the terminal device. The computer readable storage medium may also be used to temporarily store data that has been or will be output.

Those of ordinary skill in the art may realize that the example modules and algorithm steps described in conjunction with the implementations disclosed herein can be implemented by electronic hardware, computer software, or a combination of the two, in order to clearly explain the hardware and software interchangeability, in the above description, the composition and steps of each example have been generally described according to function. Whether these functions are executed in hardware or software depends on the specific application of the technical solution and design constraints. Professional technicians can use different methods to implement the described functions for each specific application, but such implementation should not be considered beyond the scope of this application.

Those skilled in the art can clearly understand that, for the convenience and conciseness of description, the specific working process of the above-described module can refer to the corresponding process in the foregoing method implementations, and details are not repeated herein.

In the implementations of the present disclosure, it should be understood that, the method disclosed in implementations provided herein may be implemented in other manners. For example, the device/apparatus implementations described above are merely illustrative; for instance, the division of the module is only a logical function division and there can be other manners of division during actual implementations, for example, multiple modules (units) or assemblies may be combined or may be integrated into another system, or some features may be ignored, omitted, or not performed. In addition, coupling or communication connection between each illustrated or discussed component may be direct coupling or communication connection, or may be indirect coupling or communication among devices or modules via some interfaces, and may be electrical connection or other forms of connection.

The units described as separate components may or may not be physically separated, the components illustrated as units may or may not be physical units, that is, they may be in the same place or may be distributed to multiple network elements. Part or all of the units may be selected according to actual needs to achieve the purpose of the technical solutions of the implementations.

In addition, the functional units in various implementations of the present disclosure may be integrated into one processing unit, or each unit may be physically present, or two or more units may be integrated into one unit. The above-mentioned integrated unit can be implemented in the form of hardware or a software function unit.

The integrated unit may be stored in a computer readable memory when it is implemented in the form of a software functional unit and is sold or used as a separate product. Based on such understanding, the technical solutions of the present disclosure essentially, or the part of the technical solutions that contributes to the related art, or all or part of the technical solutions, may be embodied in the form of a software product which is stored in a memory and includes instructions for causing a computer device (which may be a personal computer, a server, or a network device and so on) to perform all or part of the operations described in the various implementations of the present disclosure. The memory includes various medium capable of storing program codes, such as a universal serial bus (USB), a ROM, a RAM, a removable hard disk, Disk, compact disc (CD), or the like.

## Claims

1. A method for medical service management, being applicable to a blockchain node device and comprising:
receiving a registration request of at least one related party, wherein the at least one related party at least comprises one of a doctor, a nurse, an auxiliary person, a medical-equipment provider, and a medical-equipment manager;
for each of the at least one related party:
determining, according to the registration request, a medical service that the related party needs to participate in; and
determining a sharing ratio for the related party according to the medical service that the related party needs to participate in;
receiving (S101) an appointment request from a user, the appointment request comprising a user identity;
searching and recommending (S102) medical service information according to conditions set by the user and the appointment request, the medical service information comprising recommended medical-staff information, recommended medical service type information, and recommended drug information; wherein recommending the medical service information according to the appointment request comprises:
obtaining medical record information of the user according to the appointment request;
obtaining one or more groups of sorted medical-staff information by sorting one or more pieces of medical-staff information according to the medical record information of the user; and
receiving one group of medical-staff information selected by the user from the one or more groups of sorted medical-staff information, and determining the recommended medical-staff information, wherein
the recommended medical-staff information comprises following information of a medical staff: an identity, department information, skill certification information, length of service information, occupation information, and medical experience information;
determining (S103) a medical service scheme according to the medical service information;
generating (S104) a medical-fee payment contract according to the medical service scheme; and
transferring (S105), according to the medical-fee payment contract and the sharing ratio, medical fee from a digital wallet of the user to at least one digital wallet of the at least one related party of the medical service scheme, in response to receiving from the user a confirmation message of the medical-fee payment contract.

2. The method of claim 1, further comprising the following after receiving the appointment request from the user:
verifying personal information of the user according to the user identity.

3. The method of claim 2, wherein the personal information comprises at least one of personal medical insurance information and personal medical record information, and verifying the personal information of the user according to the user identity comprises:
obtaining, according to the user identity, at least one of the personal medical insurance information and the personal medical record information; and
obtaining a verification result of at least one of the personal medical insurance information and the personal medical record information.

4. The method of claim 1, wherein the confirmation message is a message for confirming that the medical service scheme is correct, or a message for confirming of an updated medical service scheme.

5. The method of claim 4, wherein transferring, according to the medical-fee payment contract, the medical fee from the digital wallet of the user to the at least one digital wallet of the at least one related party of the medical service scheme comprises:
for each of the at least one related party of the medical service scheme:
obtaining a sharing ratio corresponding to the related party; and
transferring, according to the sharing ratio obtained, the medical fee to a digital wallet of the related party of the medical service scheme.

6. A blockchain node device, comprising:
a processor (301);
an input device (302);
an output device (303); and
a memory (304) storing program instructions;
the processor, the input device, the output device, and the memory being coupled with each other; and
the program instructions, when executed by the processor, causes the processor to execute the method of any of claims 1 to 5.

7. A computer readable storage medium, storing computer programs, the computer program comprising program instructions, which when executed by a processor of an electronic device, causes the processor to execute the method of any of claims 1 to 5.

## Patentansprüche

1. Verfahren für die Verwaltung medizinischer Dienste, das auf eine Blockchain-Knotenvorrichtung anwendbar ist und Folgendes umfasst:
Empfangen einer Registrierungsanfrage von wenigstens einer zugehörigen Partei, wobei die wenigstens eine zugehörige Partei wenigstens eines von einem Arzt, einer Krankenschwester, einer Hilfsperson, einem Anbieter für medizinische Ausrüstung und einem Verwalter für medizinische Ausrüstung umfasst;
für jede der wenigstens einen zugehörigen Partei:
Bestimmen eines medizinischen Dienstes, an dem die zugehörige Partei teilnehmen muss, gemäß der Registrierungsanfrage; und
Bestimmen eines Aufteilungsverhältnisses für die zugehörige Partei gemäß dem medizinischen Dienst, an der die zugehörige Partei teilnehmen muss;
Empfangen (S101) einer Terminanfrage von einem Benutzer, wobei die Terminanfrage eine Benutzeridentität umfasst;
Suchen und Empfehlen (S 102) von Informationen über medizinische Dienste gemäß den von dem Benutzer eingestellten Bedingungen und der Terminanfrage, wobei die Informationen über medizinische Dienste Informationen über empfohlenes medizinisches Personal, Informationen über die empfohlene Art der medizinischen Dienste und Informationen über empfohlene Arzneimittel umfassen; wobei das Empfehlen der Informationen über medizinische Dienste gemäß der Terminanfrage Folgendes umfasst:
Erhalten von Informationen über die Krankenakte des Benutzers gemäß der Terminanfrage;
Erhalten einer oder mehrerer Gruppen von sortierten Informationen über medizinisches Personal durch Sortieren eines oder mehrerer Teile von Informationen über medizinisches Personal gemäß den Krankenakteninformationen des Benutzers; und
Empfangen einer Gruppe von Informationen über medizinisches Personal, die von dem Benutzer aus der einen oder den mehreren Gruppen von sortierten Informationen über medizinisches Personal ausgewählt wurde, und Bestimmen der Informationen über empfohlenes medizinisches Personal, wobei
die Informationen über das empfohlene medizinische Personal Folgende Informationen über ein medizinisches Personal umfassen: eine Identität, Informationen über die Abteilung, Informationen über die Fähigkeitszertifizierung, Informationen über die Dauer des Dienstes, Informationen über den Beruf und Informationen über die medizinische Erfahrung;
Bestimmen (S103) eines Schemas für medizinische Dienste gemäß den Informationen über die medizinische Dienste;
Erzeugen (S104) eines Vertrags über die Bezahlung medizinischer Gebühren gemäß dem Schema für medizinische Dienste; und
Übertragen (S105) der medizinischen Gebühr von einer digitalen Brieftasche des Benutzers an wenigstens eine digitale Brieftasche der wenigstens einen zugehörigen Partei des Schemas für medizinische Dienste gemäß dem Vertrag über die Bezahlung medizinischer Gebühren und dem Aufteilungsverhältnis als Reaktion auf das Empfangen einer Bestätigungsnachricht des Vertrags über die Bezahlung medizinischer Gebühren von dem Benutzer.

2. Verfahren nach Anspruch 1, das nach dem Empfangen der Terminanfrage von dem Benutzer ferner Folgendes umfasst:
Überprüfen der persönlichen Informationen des Benutzers gemäß der Benutzeridentität.

3. Verfahren nach Anspruch 2, wobei die persönliche Informationen wenigstens eines von persönlichen Krankenversicherungsinformationen und persönlichen Krankenakteninformationen umfassen, und das Überprüfen der persönlichen Informationen des Benutzers gemäß der Benutzeridentität Folgendes umfasst:
Erhalten von wenigstens einer der persönlichen Krankenversicherungsinformationen und der persönlichen Krankenakteninformationen gemäß der Benutzeridentität; und
Erhalten eines Überprüfungsergebnisses von wenigstens einer der persönlichen Krankenversicherungsinformationen und der persönlichen Krankenakteninformationen.

4. Verfahren nach Anspruch 1, wobei die Bestätigungsnachricht eine Nachricht zum Bestätigen, dass das Schema für medizinische Dienste korrekt ist, oder eine Nachricht zum Bestätigen eines aktualisierten Schemas für medizinische Dienste ist.

5. Verfahren nach Anspruch 4, wobei das Übertragen der medizinischen Gebühr von der digitalen Brieftasche des Benutzers zu der wenigstens einen digitalen Brieftasche der wenigstens einen zugehörigen Partei des Schemas für medizinische Dienste gemäß dem Vertrag für die Bezahlung medizinischer Gebühren Folgendes umfasst:
für jede der wenigstens einen zugehörigen Partei des Schemas für medizinische Dienste:
Erhalten eines Aufteilungsverhältnisses, das der zugehörigen Partei entspricht; und
Übertragen der medizinischen Gebühr auf eine digitale Brieftasche der zugehörigen Partei des Schemas für medizinische Dienste gemäß dem erhaltenen Aufteilungsverhältnis.

6. Blockchain-Knotenvorrichtung, die Folgendes umfasst:
einen Prozessor (301);
eine Eingabevorrichtung (302);
eine Ausgabevorrichtung (303); und
einen Arbeitsspeicher (304), der Programmanweisungen speichert;
wobei der Prozessor, die Eingabevorrichtung, die Ausgabevorrichtung und der Arbeitsspeicher miteinander gekoppelt sind; und
die Programmanweisungen, wenn sie von dem Prozessor ausgeführt werden, den Prozessor veranlassen, das Verfahren nach einem der Ansprüche 1 bis 5 auszuführen.

7. Computerlesbares Speichermedium, das Computerprogramme speichert, wobei das Computerprogramm Programmanweisungen umfasst, die, wenn sie von einem Prozessor einer elektronischen Vorrichtung ausgeführt werden, den Prozessor veranlassen, das Verfahren nach einem der Ansprüche 1 bis 5 auszuführen.

## Revendications

1. Procédé de gestion de service médical, étant applicable à un dispositif de nœud de chaîne de blocs et comprenant :
la réception d'une demande d'enregistrement d'au moins une partie concernée, dans laquelle l'au moins une partie concernée comprend au moins un médecin, un infirmier, une personne auxiliaire, un fournisseur d'équipement médical et un gestionnaire d'équipement médical ;
pour chacune de l'au moins une partie concernée :
la détermination, selon la demande d'enregistrement, d'un service médical auquel la partie concernée doit participer ; et
la détermination d'un ratio de partage pour la partie concernée en fonction du service médical auquel la partie concernée doit participer ;
la réception (S101) d'une demande de rendez-vous d'un utilisateur, la demande de rendez-vous comprenant une identité d'utilisateur ;
la recherche et la recommandation (S102) d'informations sur un service médical (S102) selon des conditions définies par l'utilisateur et la demande de rendez-vous, les informations sur un service médical comprenant des informations recommandées sur le personnel médical, des informations recommandées sur le type de service médical, et des informations recommandées sur des médicaments ; dans lequel la recommandation d'informations sur le service médical selon la demande de rendez-vous comprend :
l'obtention d'informations de dossier médical de l'utilisateur selon la demande de rendez-vous ;
l'obtention d'un ou de plusieurs groupes d'informations triées sur le personnel médical en triant un ou plusieurs éléments d'information sur le personnel médical selon les informations de dossier médical de l'utilisateur ; et
la réception d'un groupe d'informations sur le personnel médical sélectionné par l'utilisateur parmi les un ou plusieurs groupes d'informations triées sur le personnel médical, et la détermination des informations recommandées sur le personnel médical, dans lequel
les informations recommandées sur le personnel médical comprennent les informations suivantes d'un personnel médical : une identité, des informations sur un département, des informations sur la certification de compétences, des informations sur la durée de service, des informations sur la profession, et des informations sur l'expérience médicale ;
la détermination (S103) d'un schéma de service médical selon les informations sur le service médical ;
la génération (S104) d'un contrat de paiement de frais médicaux selon le régime de service médical ; et
le transfert (S105), selon le contrat de paiement de frais médicaux et le ratio de partage, des frais médicaux d'un portefeuille numérique de l'utilisateur à au moins un portefeuille numérique de l'au moins une partie concernée du régime de service médical, en réponse à la réception de l'utilisateur d'un message de confirmation du contrat de paiement des frais médicaux.

2. Procédé selon la revendication 1, comprenant en outre ce qui suit après la réception de la demande de rendez-vous de l'utilisateur :
la vérification d'informations personnelles de l'utilisateur selon l'identité de l'utilisateur ;

3. Procédé selon la revendication 2, dans lequel les informations personnelles comprennent au moins des informations parmi des informations personnelles d'assurance médicale et des informations personnelles de dossier médical, et la vérification des informations personnelles de l'utilisateur selon l'identité d'utilisateur comprend :
l'obtention, selon l'identité de l'utilisateur, au moins d'informations parmi des informations personnelles d'assurance médicale et des informations personnelles de dossier médical; et
l'obtention d'un résultat de vérification au moins d'informations parmi des informations personnelles d'assurance médicale et des informations personnelles de dossier médical.

4. Procédé selon la revendication 1, dans lequel le message de confirmation est un message destiné à confirmer que le programme de service médical est correct, ou un message destiné à confirmer un programme de service médical mis à jour.

5. Procédé selon la revendication 4, dans lequel le transfert, selon le contrat de paiement de frais médicaux, des frais médicaux du portefeuille numérique de l'utilisateur à l'au moins un portefeuille numérique de l'au moins une partie concernée du régime de service médical comprend :
pour chacune de l'au moins une partie concernée du régime de service médical :
l'obtention d'un ratio de partage correspondant à la partie concernée ; et
le transfert, selon le ratio de partage obtenu, des frais médicaux à un portefeuille numérique de la partie concernée du régime de service médical.

6. Dispositif de nœud de chaîne de blocs, comprenant :
un processeur (301) ;
un dispositif d'entrée (302) ;
un dispositif de sortie (303) ; et
une mémoire (304) stockant des instructions de programme ;
le processeur, le dispositif d'entrée, le dispositif de sortie, et la mémoire étant couplés les uns aux autres; et
les instructions de programme, lorsqu'elles sont exécutées par le processeur, amènent le processeur à exécuter le procédé selon l'une quelconque des revendications 1 à 5.

7. Support de stockage lisible par ordinateur, stockant des programmes d'ordinateur, le programme d'ordinateur comprenant des instructions de programme, qui, lorsqu'elles sont exécutées par un processeur d'un dispositif électronique, amènent le processeur à exécuter le procédé selon l'une quelconque des revendications 1 à 5.
